# EUROPEAN PATENT SPECIFICATION

(11) **EP 3 723 665 B1**
(45) Date of publication and mention of the grant of the patent: **11.08.2021**
(21) Application number: 18830747.4
(22) Date of filing: 12.12.2018
(51) Int. Cl.: A61F 2/24

(54) **CRIMPING DEVICE FOR COLLAPSIBLE VALVES**
CRIMPVORRICHTUNG FÜR FALTBARE KLAPPEN
DISPOSITIF DE SERTISSAGE POUR VALVES PLIABLES

(30) Priority: 13.12.2017 EP 17207035
(43) Date of publication of application: 21.10.2020
(73) Proprietor: Epygon, 13100 Aix-en-Provence (FR)
(72) Inventor: PASQUINO, Enrico, 1073 Savigny (CH); SCORSIN, Marcio, 2213 Luxembourg (LU); VALERIO, Lorenzo, 31010 Moriago della Battaglia (IT); MARCHISIO, Andrea, 10015 Ivrea (IT); GASTALDIN, Davide, 15040 Valmacca (IT); PASQUINO, Stefano, 1073 Savigny (CH)
(74) Representative: Müller, Christoph Emanuel
(86) International application number: PCT/EP2018/084505
(87) International publication number: WO 2019/115592

(56) References cited:
- EP-A1- 2 014 257
- EP-A1- 2 644 158

## Description

### Field of invention

The present invention relates to a device dedicated to diameter reduction of collapsible valves, in particular heart valves.

### State of the art

In recent years, the development of both balloon-expandable and self-expandable valves allows crimping the valvular structure into a cylindrical shaft prior to deploy the valve in the correct anatomical position.

In the present document, the expression "crimping a valve" has to be understood as "reducing the diameter of a valve in a way as to make it possible to load it within a hollow cylindrical portion of a delivery system".

Furthermore, the terms "valve", "stent" or "stent-valve" are indifferently used in the present document.

In the present context, the stent-valve is a prosthesis since its function is to replace a native valve.

Currently on the market crimping tools provide a single stage crimping mechanism (WO 2004/019768, US 2007/0056346, US 2015/0336150). For complex devices, which include multiple anchoring system, having a single stage crimping tool may be flawed since it obliges to reduce simultaneously the entire stent frame diameter, including the anchoring system. Another option is to crimp only the main structural stent, leaving the marginal anchoring system, which protrude externally from the stent, to its original shape, and loading subsequently the crimped stent into a delivery system with a double-sleeve stent cover. Nonetheless, many projects include their anchoring system within the stent frame and it is not possible to discriminate it during the crimping procedure. In addition, since these anchors are deemed to sustain the pressure loads when prostheses are implanted, anchoring system usually offer higher resistance to radial forces, thus deforming the stent frame structures during the process of crimping. In this context, having the possibility of acting on selected portion of the stent during crimping phase may positively affect the outcomes of the procedure itself. In fact, crimping annular portion of the stent to its final diameter without impacting directly to external anchoring protrusion of stent frames may reduce the stresses imparted to the structures, thus leading to a more homogenous and safe crimping process.

Crimping devices acting on selected portion of the valves are disclosed in the following patent documents : EP 2 014 257 A1 and EP 2 644 158 A1.

In those devices each actuator has a bar shape that is radially oriented, i.e. perpendicular with respect to the external surface of the cylindrical unit.

Those prior art devices are cumbersome and may not allow to obtain a precise crimping action.

There is therefore a need to improve the existing crimping devices.

### General description of the invention

The present invention consists in an improved crimping tool that allows the conditioning of collapsible valves, via a reduction of the stent diameter. The stent is compressed so that it can be loaded e.g. into a valve-cover of a delivery system.

More precisely the invention relates to a crimping device for reducing the diameter of collapsible valves, said device including at least two independent cylindrical crimping units that are each adapted to crimp a different specific part of a valve, independently from the other crimping unit(s), each of said unit comprising a diaphragm rotation crimping mechanism that includes crimping jaws, and wherein each unit comprises an actuator for activating said mechanism, characterized by the fact that said actuator is a handling screw that is tangentially fixed to the external surface of the cylindrical crimping unit.

The use of the handling screw(s) according to the invention provide several advantages with respect to the prior art devices, in particular a volume reduction of the crimping device and a more precise crimping, i.e. the different diameters of the collapsed can be more precisely defined.

In a preferred embodiment, the device is made for the crimping of collapsible heart valves. To this effect, it comprises three crimping units that are adapted to independently act over atrial, annular and ventricular portions of the stent. This allows to reduce the stent-valve annular diameter without affecting both atrial and ventricular stent portions. In particular, with the crimping device of the invention, it is possible to act on the ventricular portion of the stent that may comprise an anchoring system, after the crimping annular portion. Mutual and simultaneous stresses over the whole stent, that may affect its general structure, are then avoided. Finally, the ability of leaving both atrial and ventricular portions of the stent protruding outwards the crimping jaws, offers the possibility to load the stent within the delivery system by connecting elements such as atrial flaps or ventricular engagement bodies to other parts of the delivery system.

### Detailed description of the invention

The invention will be better understood in the present chapter, with non-limiting examples illustrated by the following figures:

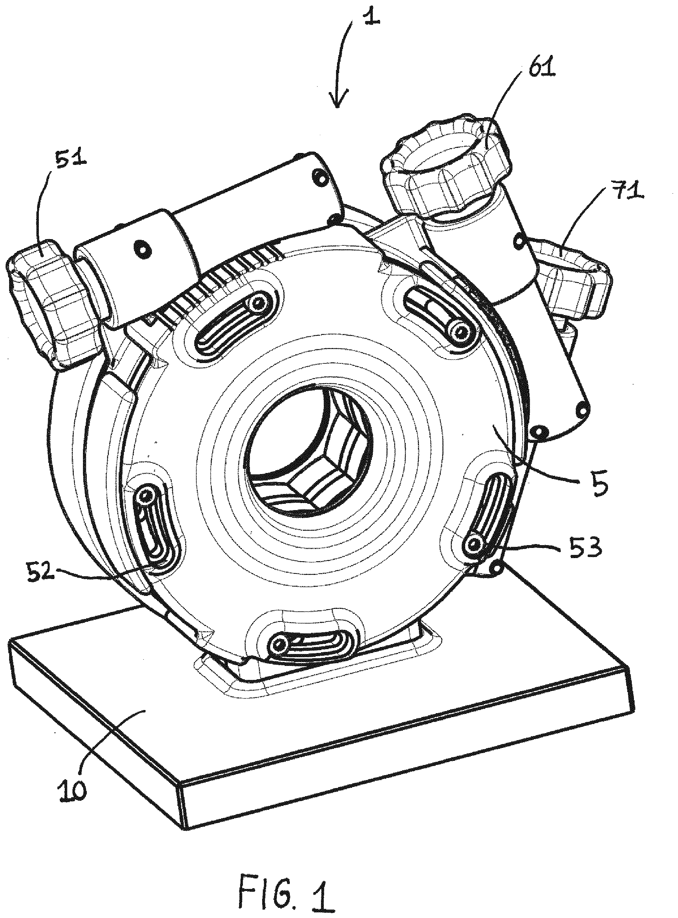

Figure 1: Lateral view of the three-stage crimping device. Three separated screws provide independent crimping movements using this device.
Figure 2: Sectional view of the crimper. From this perspective, it is possible to discriminate the three independent crimping units 2, 3 and 4 made by multiple inclined adjoining jaws.
Figure 3 - A: Lateral view of the crimping device with partial closure of atrial crimping unit 2. In this configuration, jaws are partially rotated so that crimping system is only marginally closed.

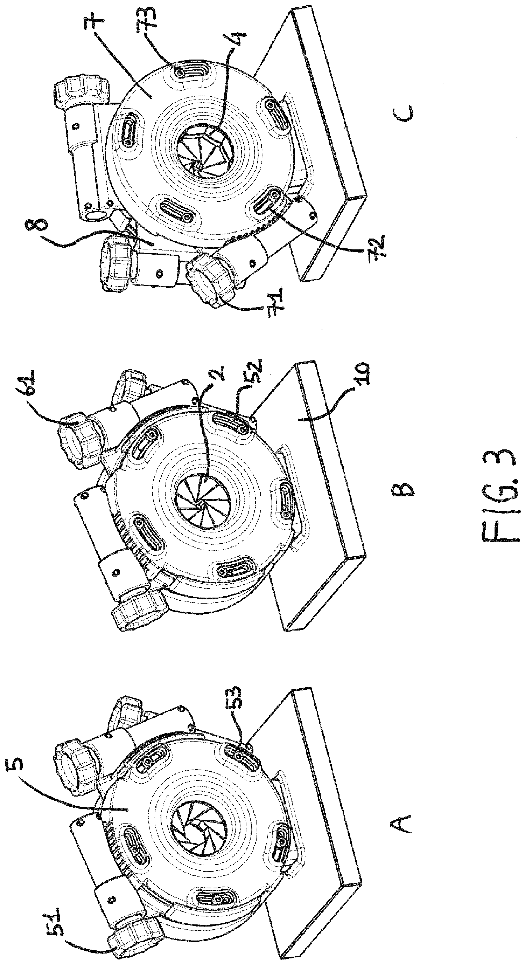

Figure 3 - B: Lateral view of the crimping device with total closure of atrial crimping unit 2. In this configuration, jaws are rotated until final positioning of control screw 53.
Figure 3 - C: Lateral view of the crimping device from ventricular crimping unit side. It appears evident as the three different crimping units are independent. In fact, despite atrial unit appears totally closed, both annular and ventricular crimping units are in their fully-open configuration.

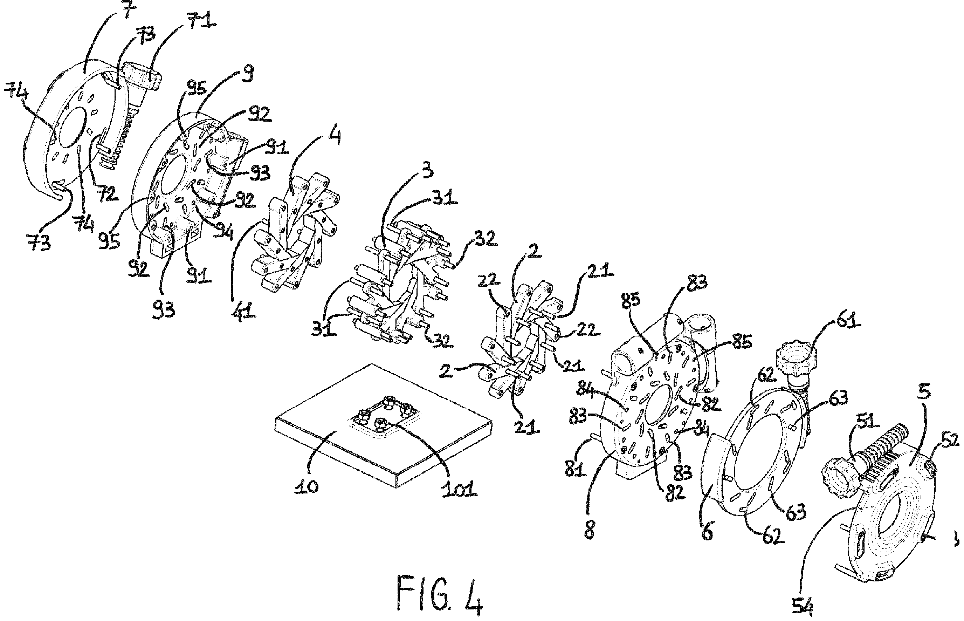

Figure 4: Exploded later view of each components of the three-stage crimping device.

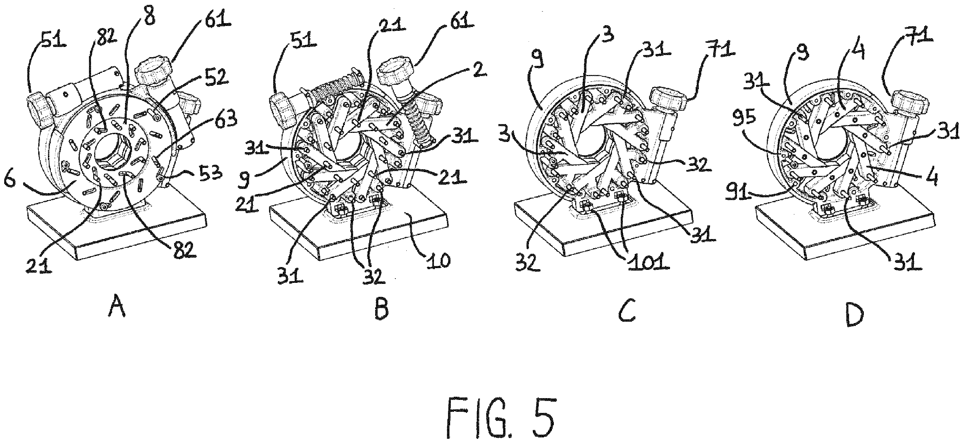

Figure 5 - A: Perspective view of the crimping device without the atrial external cam 5.
Figure 5 - B: Perspective view of the crimping device without the atrial external cam 5, annular cam 6 and atrial part of the anterior inner translational shell 8.
Figure 5 - C: Perspective view of the crimping device without the atrial external cam 5, annular cam 6, anterior inner translational shell 8 and the atrial crimping mechanism 2, 21.
Figure 5 - D: Perspective view of the crimping device without the atrial external cam 5, annular cam 6, anterior inner translational shell 8, the atrial crimping mechanism 2, 21 and the annular crimping mechanism 3, 32.

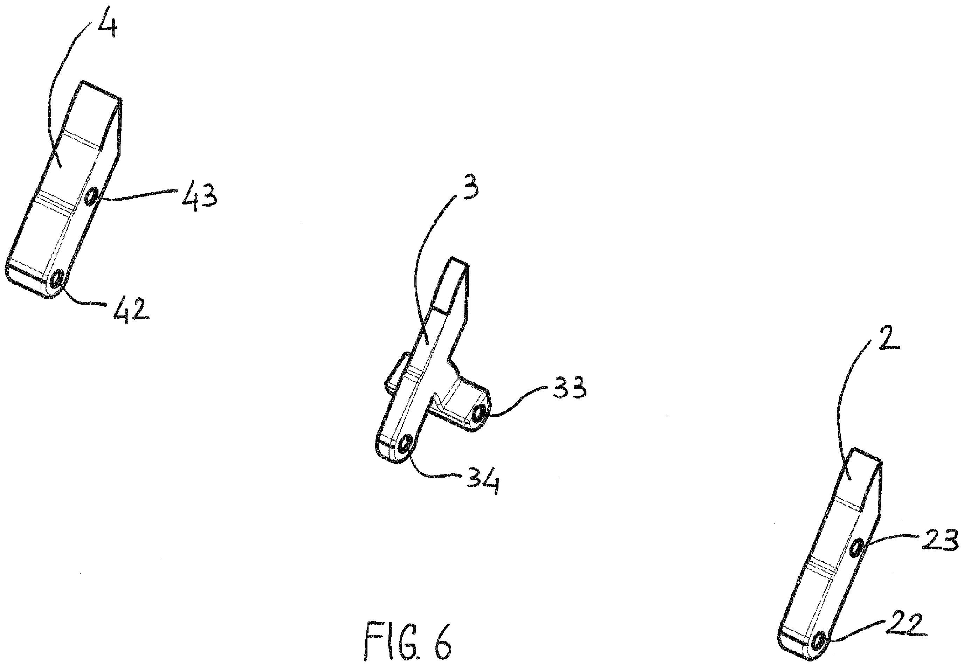

Figure 6: perspective view of a singular atrial 2, annular 3 and ventricular 4 crimping jaws with their correspondent hinge (22, 34, 42) and translation (23, 33, 43) peg holes.

### Numerical references used in the figures

- 1: three-stage crimping device
- 2: atrial crimping unit
- 3: annular crimping unit
- 4: ventricular crimping unit
- 5: atrial external cam
- 6: annular cam
- 7: ventricular external cam
- 8: anterior inner translational shell
- 9: posterior inner translational shell
- 10: support base
- 21: atrial translation pegs
- 22: atrial hinge holes
- 31: principal hinge pegs
- 32: annular translation pegs
- 41: ventricular translation pegs
- 51: atrial handling screw
- 52: atrial cam sliding guides
- 53: atrial cam scroll screws
- 54: atrial peg translation guides
- 61: ventricular handling screw
- 62: annular cam sliding guides
- 63: annular peg support guides
- 71: ventricular handling screw
- 72: ventricular cam sliding guides
- 73: ventricular cam scroll screws
- 74: ventricular peg translation guides
- 81: inner translational shell fixing screws
- 82: atrial peg support guides
- 83: annular peg support guides on the anterior inner translational shell
- 84: principal hinge peg binding holes on the anterior inner translational shell
- 85: scroll screw fixing holes of the anterior inner translational shell
- 91: principal hinge peg binding holes on the posterior inner translational shell
- 92: ventricular peg support guides
- 93: annular peg support guides on the posterior inner translational shell
- 94: annular peg support guides on the posterior inner translational shell
- 95: scroll screw fixing holes of the posterior inner translational shell
- 101: support base fixing screws

The illustrated example includes a three-stage independent crimping device 1 for collapsible valves.

An important feature of the device includes the presence of three independent crimping units 2, 3, 4 for allowing acting separately on atrial, annular or ventricular portion of a valve for reducing its diameter.

The present disclosure describes a crimping tool designed to reduce selectively and independently different stent portion using a diaphragm rotation mechanism. The crimper device includes an atrial external cam 5 and a ventricular external cam 7 which respectively allow the rotational movements of atrial 2 and ventricular 4 crimping jaws. Moreover, an internal annular cam 7 allows the movement of the annular crimping unit 3. Atrial, annular and ventricular jaws, located within the inner translational shell 8-9, have different thickness, depending of the length of the correspondent stent portions in totally crimped configuration. Both external atrial and ventricular cams 5,7, annular cam 6, inner translational shell 8-9 and crimping jaw system 2-3-4 present circular shape, with a center radial hole allowing the insertion of the stent frame during crimping procedure. Actual movements of jaws, which provide crimping effect, are based on rotational movement of each unit jaws around a principal hinge peg (one for each adjacent jaw). The mutual contact between adjacent jaws and their inner portion surface design (in contact with the stent), allow to transform the rotation of the jaw around their hinge pegs in the progressive reduction of inner diameter of crimping units. This crimping mechanism is mediated by guided movement of appropriate translational pegs, fixed to each jaw, within their correspondent sliding guides (one for each adjacent jaw). Actuation movement, that will be subsequently described, is conducted using an handling screw 51, 61, 71. In addition, the present device may include different separate actuators, each one dedicated to each single crimping unit or a singular actuator able to selectively act on each different crimping unit. Such unique actuator may be manufactured so that it will be possible to progressively crimp down the annular, ventricular and atrial stent section in sequence. Crimping unit actuation sequence may be decided independently by the user.

Fig.6 shows a clear representation of crimping jaws. As visible, each atrial, annular and ventricular crimping jaw has two holes. External holes in atrial 22, annular 34 and ventricular 42 crimping jaw allow the passage of the principal hinge peg 31 around which each jaw rotate during crimping process. Proximal holes 23 - 33 - 43 allocated on the atrial, annular and ventricular crimping jaws allow the passage of the correspondent pegs that ensure the jaws rotation during crimping. Crimping jaws, which represent the only portion of the crimping device in contact with the stent, may be manufactured using low friction materials, such as Delrin, Polyoxymethylene (POM), ultra-high-molecular-weight polyethylene (UHMWPE), Nylon, or polytetrafluoroethylene (PTFE). External atrial and ventricular cams 5 - 7, annular cam 6, inner translational shell 8 - 9, support base 10, fixing screws 81 - 101, scroll screws 53 - 73, hinge pegs 31 and translational pegs 21 - 32- 41 may be manufactured with blood-resistance materials, such as plastic (polyester material, polyethylene terephthalate PET, polyvinyl chloride PVC etc.) or metal (stainless steel AISI 316, corrosion-resistant aluminum).

Fig. 5 A-D shows the crimping device while removing progressively crimping elements from the external atrial cam 5-A until leaving only the ventricular crimping mechanism 5-D. To better understand the complexity of the crimping device, please refers to Fig. 4, where an exploded view of each element of the crimping device is reported. Once stent is inserted in central opening of the crimper device and correctly aligned so that each atrial, annular and ventricular portion of the stent are aligned to its correspondent crimping unit of the device, it is possible to proceed with crimping procedure.

Crimping procedure description for each atrial, annular and ventricular unit will be separately described hereafter.

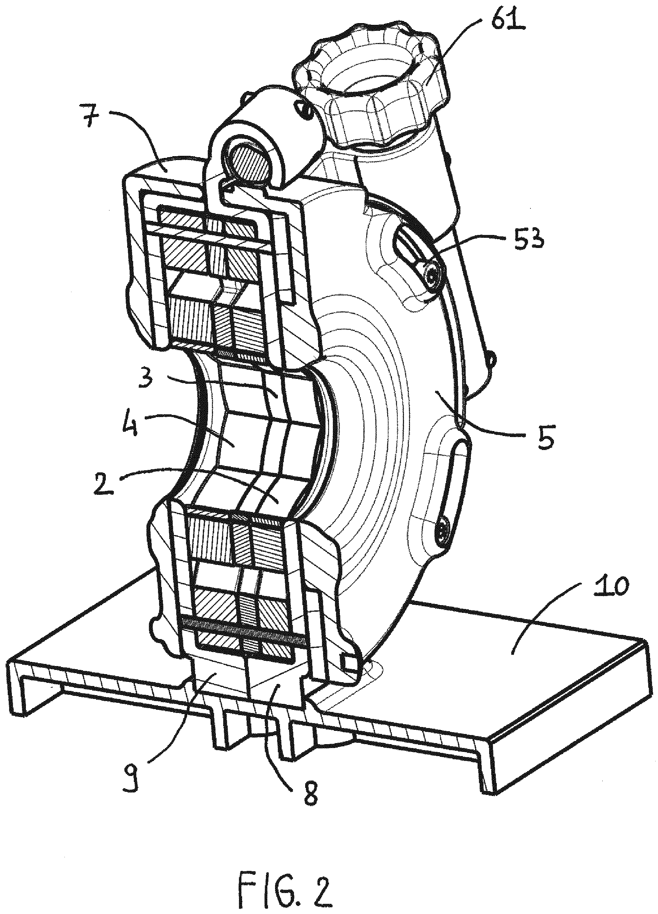

Atrial cam presents four main elements: a structural shell 5 where an atrial handling screw 51 can be screwed, multiple atrial cam sliding guides 52 and multiple atrial peg translation guides 54. Atrial cam scroll screws 53 are inserted within the sliding guides and fixed to the anterior inner translational shell 85. Minimum crimping diameter of atrial portion is related to atrial cam sliding guides length. In fact, when in fully-open configuration, atrial cam scroll screws 53 are in contact with one end of the atrial cam sliding guides 52 (as in Fig. 2). For crimping atrial portion of the stent, it is necessary to act on atrial handling screw 51 located on the atrial cam 5. In this configuration, screwing the handling screw within its compartment on the atrial cam, it is possible to rotate the cam shell itself. Such rotational movement allows each atrial jaw 2, in mutual contact with its adjacent jaw, while rotating around its principal hinge peg 31, to reduce the inner diameter of the atrial crimping unit. Atrial pegs translational guides 54, located on the inner surface of the atrial cam, force the radial movements of the atrial pegs 21, fixed to each atrial jaw 2, while rotating the atrial cam. Atrial pegs movement within the translational guides 54 is aided by the atrial peg support guides 82 located on the anterior inner translational shell 8. Since the atrial cam scroll screws 53 are fixed to the translational shell 8, the rotation of the atrial cam, as well as the progressive reduction of the crimping diameter imparted by the crimping system, is allowed until the scroll screws 53 touch the opposite end of the atrial cam sliding guides.

Using the same functioning principle of the atrial crimping procedure, it is possible to act on the device for the stent annular crimping section. Annular cam apparatus, which provide the rotational movements for the crimping process, consists in a structural shell 6, onto which it acts an annular handling screw 61. Moreover, on the annular cam are present multiple annular cam sliding guides 62 and annular peg support guides 63. Minimum crimping diameter of annular portion is related to annular cam sliding guides length. Screwing the handling screw 61 within its compartment on the annular cam 6, it is possible to rotate the cam shell itself. Such rotational movement allows all annular jaws 3, that are in mutual contact with each other's, while rotating around their principal hinge peg 31, to reduce the inner diameter of the annular crimping unit. Annular peg translational guides 83 - 93, located on both anterior and posterior portions of the inner translational shell 8 - 9, force the radial movements of the annular pegs 32, fixed to each annular jaw, while rotating the annular cam 6. Annular pegs movement within the translational guides 83 - 93 is aided by the annular peg support guides 63 located on the annular cam 6. As for atrial section crimping procedure, since the atrial cam scroll screws 53 are fixed to the translational shell 8, the rotation of the annual cam, as well as the progressive reduction of the crimping diameter imparted by the jaws crimping system, is allowed until the scroll screws 53 touch one end of the annular cam sliding guides 62.

The crimping procedure of the ventricular portion of the stent may be implemented separately after the end of annular crimping procedure alone or after both annular and atrial crimping procedures. It is important to underline as it may be possible to act on each crimping unit independently using the present device.

Ventricular cam presents four main elements: a structural shell 7 where the ventricular handling screw 71 can be screwed, multiple ventricular cam sliding guides 72 and multiple ventricular peg translation guides 74. Ventricular cam scroll screws 73 are inserted within their correspondent sliding guides 72 and fixed to the posterior inner translational shell 95. Minimum crimping diameter of ventricular portion is related to ventricular cam sliding guides length. In fact, when in fully-open configuration, ventricular cam scroll screws 73 are in contact with one end of the ventricular cam sliding guides 72 (as in Fig. 3 - C). For crimping ventricular portion of the stent, it is necessary to act on ventricular handling screw 71 located on the ventricular cam 7. Such rotative actuation movement may be substituted by an electric, lever or other mechanical actuations, as for previous crimping mechanisms described. Screwing the handling screw within its compartment on the ventricular cam, it is possible to rotate the cam shell. Such rotational movement allows each ventricular jaw 4, in mutual contact with its adjacent jaw, while rotating around its principal hinge peg 31, to reduce the inner diameter of the ventricular crimping unit. Ventricular pegs translational guides 74, located on the inner surface of the ventricular cam 7, force the radial movements of the ventricular pegs 41, fixed to each jaw 4, while rotating the ventricular cam. Ventricular pegs movement within the translational guides 74 is aided by the ventricular peg support guides 92 located on the posterior inner translational shell 9. Since the ventricular cam scroll screws 73 are fixed to the translational shell 9, the rotation of the ventricular cam, as well as the progressive reduction of the crimping diameter imparted by the jaws crimping system, is allowed until the scroll screws 73 touch the opposite end of the ventricular cam sliding guides 72.

Atrial, annular and ventricular crimping mechanisms (2 - 3 - 4), including correspondent hinge and translational pegs (21, 31, 32 and 41) are mutually interconnected and compenetrated. In addition, all these elements are located within the inner translational shell 8 - 9. Anterior and posterior Inner translational shells 8 - 9, bonded together using appropriate fixing screws 81, form the inner translational shell. The latter is a not moving element, which provides support during crimping phase and allows fixing the crimping structures to the support base 10 via support base fixing screws 101. The innovative design of this three-stage crimping device allows obtain a complex and very versatile tool without loss in compactness and ease of use.

## Claims

1. Crimping device (1) for reducing the diameter of collapsible valves, said device (1) including at least two independent cylindrical crimping units (2,3,4) that are each adapted to crimp a different specific part of a valve, independently from the other crimping unit(s), each of said unit (2,3,4) comprising a diaphragm rotation crimping mechanism that includes crimping jaws, and wherein each unit (2,3,4) comprises an actuator for activating said mechanism, **characterized by** the fact that said actuator is a handling screw (51,61,71) that is tangentially fixed to the external surface of the cylindrical crimping unit (2,3,4).

2. Crimping device (1) according to claim 1 for collapsible valves and comprising three crimping units (2,3,4), namely an atrial unit, an annular unit and a ventricular unit.

3. Crimping device (1) according to claim 1 or 2 wherein each unit (2,3,4) comprises an annular cam (5,6,7) for the rotational movements of the crimping jaws.

4. Crimping device (1) according to anyone of the previous claims wherein said crimping units (2,3,4) have different thicknesses.

5. Crimping device (1) according to anyone of the previous claims in which mutual contact between adjacent jaws and their inner portion surface design in contact with the valve allow to transform the rotation of each jaw around a hinge peg in the progressive reduction of inner diameter of the corresponding crimping unit (2,3,4).

## Patentansprüche

1. Crimpvorrichtung (1) zum Verringern des Durchmessers von kollabierbaren Ventilen, wobei die Vorrichtung (1) mindestens zwei unabhängige zylindrische Crimpeinheiten (2, 3, 4) aufweist, die jeweils geeignet sind, einen anderen spezifischen Teil eines Ventils unabhängig von der/den anderen Crimpeinheit(en) zu crimpen, wobei jede der Einheiten (2, 3, 4) einen Diaphragma-Rotations-Crimpmechanismus aufweist, der Crimpbacken enthält, und wobei jede Einheit (2, 3, 4) einen Aktuator zum Aktivieren des Mechanismus aufweist, **dadurch gekennzeichnet, dass** der Aktuator eine Handhabungsschraube (51, 61, 71) ist, die tangential an der Außenfläche der zylindrischen Crimpeinheit (2, 3, 4) befestigt ist.

2. Crimpvorrichtung (1) nach Anspruch 1 für kollabierbare Ventile und umfassend drei Crimpeinheiten (2, 3, 4), nämlich eine atriale Einheit, eine ringförmige Einheit und eine ventrikuläre Einheit.

3. Crimpvorrichtung (1) nach Anspruch 1 oder 2, wobei jede Einheit (2, 3, 4) einen ringförmigen Nocken (5, 6, 7) für die Drehbewegungen der Crimpbacken aufweist.

4. Crimpvorrichtung (1) nach einem der vorhergehenden Ansprüche, wobei die Crimpeinheiten (2, 3, 4) unterschiedliche Dicken aufweisen.

5. Crimpvorrichtung (1) nach einem der vorhergehenden Ansprüche, bei der der gegenseitige Kontakt zwischen benachbarten Backen und die Gestaltung ihrer Innenfläche in Kontakt mit dem Ventil es ermöglichen, die Drehung jeder Backe um einen Scharnierzapfen in die fortschreitende Verringerung des Innendurchmessers der entsprechenden Crimpeinheit (2, 3, 4) umzuwandeln.

## Revendications

1. Dispositif de crimping (1) pour réduire le diamètre de valves compressibles, ledit dispositif (1) comprenant au moins deux unités de crimping cylindriques indépendantes (2, 3, 4) qui sont chacune adaptées pour crimper une partie spécifique différente d'une valve, indépendamment de l'autre ou des autres unités de crimping, chacune desdites unités (2, 3, 4) comprenant un mécanisme de crimping par rotation du diaphragme qui comprend des parties de crimping, et dans lequel chaque unité (2, 3, 4) comprend un actionneur pour activer ledit mécanisme, **caractérisé par le fait que** ledit actionneur est une vis de manipulation (51, 61, 71) qui est fixée tangentiellement à la surface externe de l'unité de crimping cylindrique (2, 3, 4).

2. Dispositif de crimping (1) selon la revendication 1 pour valves compressibles et comprenant trois unités de crimping (2, 3, 4), à savoir une unité auriculaire, une unité annulaire et une unité ventriculaire.

3. Dispositif de crimping (1) selon la revendication 1 ou 2, dans lequel chaque unité (2, 3, 4) comprend une came annulaire (5, 6, 7) pour les mouvements de rotation des parties de crimping.

4. Dispositif de crimping (1) selon l'une quelconque des revendications précédentes, dans lequel lesdites unités de crimping (2, 3, 4) ont des épaisseurs différentes.

5. Dispositif de crimping (1) selon l'une quelconque des revendications précédentes dans lequel le contact mutuel entre des parties adjacentes et la conception de leur surface de partie interne en contact avec la valve permettent de transformer la rotation de chaque partie autour d'une goupille de pivot en la réduction progressive du diamètre interne de l'unité de crimping correspondante (2,3,4).
